# EUROPEAN PATENT SPECIFICATION

(11) **EP 0 980 233 B1**
(45) Date of publication and mention of the grant of the patent: **23.11.2005**
(21) Application number: 98918235.7
(22) Date of filing: 16.04.1998
(51) Int. Cl.: A61K 6/00

(54) **METHOD AND COMPOSITION FOR ADHERING TO TOOTH STRUCTURE**
VERFAHREN UND ZUSAMMENSETZUNG ZUR HAFTUNG AN ZAHNSTRUKTUREN
PROCEDE ET COMPOSITION ADHERANT A UNE STRUCTURE DENTAIRE

(30) Priority: 16.04.1997 US 43920
(43) Date of publication of application: 23.02.2000
(73) Proprietor: DENTSPLY INTERNATIONAL, INC., York, PA 17405-0872 (US)
(72) Inventor: SANG, Junjie, Magnolia, DE 19962-1482 (US)
(74) Representative: Wächtershäuser, Günter
(86) International application number: PCT/US1998/007572
(87) International publication number: WO 1998/046196

(56) References cited:
- EP-A- 0 321 683
- EP-A- 0 393 617
- EP-A- 0 408 357
- EP-A- 0 661 034
- WO-A-96/00558
- WO-A-96/00559

## Description

### Technical Field

The invention relates to adhesion of dental restoratives to teeth, porcelain, metals, amalgams and other dental surfaces. The invention provides a method and composition for adhering to such dental surfaces. Specifically, the inventive compositions include a (meth)acrylate adhesive component optionally containing one or more fillers, and a sulfinate salt activator component. The method according to the invention includes preparing the dental surface, applying the inventive material and then applying a dental restorative according to appropriate direct or indirect dental restoration procedures.

### Background of the Invention

EP-A 0 393 617, EP-A 0 408 357 and EP-A 0661 034 disclose polymerizable dental compositions, but said to disclose multifunctional polymerizable compounds having at least these (meth)acrylate moieties and a phosphate moiety.

It is most desirable, when filling a tooth cavity with a filling material, such as a polymerizable dental restorative, to ensure good adhesion between the tooth surrounding the cavity and the set (polymerized) filling material since there is thereby obtained a good seal between the set filling material and the tooth which prevents, or at least markedly inhibits, ingress of mouth fluids and bacteria into the filled cavity and thus prevents further decay or loss of the filling material. In order to achieve good adhesion between the filling material and the tooth tissues, enamel or dentin, it has been recommended to provide a primer or adhesive bonding layer intermediate to the filling material and surfaces of a prepared tooth. The filling material is normally an amalgam or non-amalgam materials for direct or indirect restorations.

The priming compositions heretofore known in the art, require the separate steps of applying a priming material to a prepared dental surface, followed by the application of a dental adhesive. Such a method is described for example, in U.S. Pat. No. 5,595,487 and in U.S. Pat. No. 5,554,030. In practice, it has been found that such multi-step procedures are laborious and time consuming. The dental clinician requires time effective procedures. Hence, use of these procedures has been undesirable.

A need exists therefore, for a dental composition which will simultaneously provide a priming and adhesive material intermediate to the prepared dental surface and the subsequently applied restorative material in both direct and indirect dental procedures. By "direct" it is meant a dental procedure where a tooth is prepared to receive a dental restorative composition which is formed and hardened in place. An "indirect" procedure is one wherein a dental construct such as an inlay, onlay, bridge, crown or the like is first prepared and then set into the prepared and adhesive-coated tooth.

### Objects of the Invention

It is an object of the present invention to provide a dental composition.

It is another object of the invention to provide such a composition which will enhance adhesion of a dental restorative material to a dental surface.

It is still another object of the invention to provide dental composition as above, which is applied as a mixture or as a single composition.

It is a further object of the invention to provide a method of restoring a tooth structure.

It is an additional object of the invention to provide a method as above, which does not require separate steps for applying a priming component and an adhesive component.

These and other object of the invention, which will become apparent from the disclosure and claims to follow, are carried out by the invention as hereinafter described and claimed.

### Summary of the Invention

A dental adhesive composition according to the invention comprises from about 5 to about 80 percent by weight of an adhesive or adhesive/primer component optionally containing one or more fillers, and from about 20 to about 95 percent by weight of an activator component; the adhesive component comprising at least about 35 percent by weight of a volatile organic solvent component, at least about 5 percent by weight of one or more polymerizable (meth)acrylate compounds, and an effective amount of a polymerization photoinitiator; the polymerizable (meth)acrylate compound being substantially soluble or homogeneously dispersed in said volatile organic solvent component.

The adhesive component can also comprise at least about 5% by weight of one or more (meth)acrylate compounds and an effective amount of a photoinitiator. The adhesive is a substantially homogeneous mixture of the above compounds but in the absence of said volatile organic solvent(s).

The activator component comprising from about 0.1 to about 10 percent by weight of an aromatic sulfinate salt and from about 90 to about 99.9 percent by weight of an activator component solvent. The activator component may optionally contain additives that promote self-cure and/or light cure interactions between the adhesive compositions and the restoratives.

A method according to the invention, for adhering a non-amalgam dental restorative to a dental surface, comprises the steps of:
(a) preparing the dental surface for restoration, including direct and/or indirect restoration;
(b) optionally etching the surface with an acid;
(c) applying a mixture of an adhesive component and an activator component to the prepared surface, thereby forming a coated dental surface; and,
(d) applying a non-amalgam dental restorative to the coated dental surface. It is an aspect of the invention that step (c) may include applying the adhesive component and the activator component sequentially rather than mixing them first.

A method according to the invention for adhering an amalgam dental restorative to a dental surface, comprises the steps of:
(a) preparing the surface for restoration;
(b) optionally etching the surface with an acid;
(c) applying a mixture of an adhesive component and an activator component to the prepared surface (or sequentially applying these two components), thereby forming a coated dental surface;
(d) optionally applying a chemically curable dental adhesive to said coated dental surface, wherein the chemically curable adhesive includes an oxidizing agent and a reducing agent; and,
(e) applying an amalgam dental restorative to the coated dental surface.

### Preferred Embodiments for Carrying Out the Invention

The dental adhesive composition according to the invention is a mixture of an adhesive component and an activator component. It is to be understood that by "adhesive" it is meant either an adhesive or a primer/adhesive component. As will be discussed below, the adhesive component may include a solvent (the primer/adhesive) or it may be a resin adhesive which is solvent-free. The term "adhesive" will be understood to include all such adhesive systems.

One preferred composition has from about 5 to about 80 percent by weight of the adhesive component and from about 20 to about 95 percent by weight of the activator component. The composition may also contain fluoride.

The inventive materials may be provided as a single composition, all components being packaged in one bottle for subsequent use, or as a "mixed" system wherein the adhesive component is packaged separately from the activator component, the two being mixed at the time of use and before being used in the inventive method. Both such packaging applications are within the scope of the invention.

The adhesive component preferably includes at least about 35 percent by weight of a volatile organic solvent and at least about 5 percent by weight of one or more polymerizable (meth)acrylate compounds, the remaining components to form 100 percent by weight of the adhesive component being initiators or other cure components, polymerizable monomers and oligomers, polymerizable phosphates or other ethylenically unsaturated components, other optional fillers, fluoride release compounds, stabilizers and the like. An example of a useful fluoride release compound is cetylamine hydrofluoride.

The adhesive component may optionally contain a filler including any filler that is useful in dental applications. For example, an inorganic filler such as silanated inorganic silica may be employed. Another example is a sol-gel inorganic/organic nano-scale composite. A prepolymerized organic filler may also be employed.

The adhesive component preferably includes an effective amount of a photopolymerization initiator. The polymerizable (meth)acrylate compounds should be substantially soluble or substantially homogeneously dispersed in the selected solvent or solvents, and are polymerizable to form a polymeric material. Certain filler materials may not necessarily be soluble in the solvent(s) and as such are preferably substantially homogeneously dispersed in the adhesive component.

Examples of useful volatile organic solvents for the adhesive component include ethanol, methanol, isopropanol, dimethyl ketone, ethylmethyl ketone, water, and the like, as well as mixtures thereof. The adhesive component according to the present invention may also be a solvent-free adhesive resin, as discussed above.

Preferred polymerizable methacrylate compounds include for example, those monomers having a solubility in water of less than about 5%, and more preferably have a solubility in water of less than about 1%. Other useful polymerizable (meth)acrylate compounds include multifunctional polymerizable compounds having at least three (meth)acrylate moieties and a phosphate moiety. Still further preferred monomers include surface active monomers having acid functional groups and containing (meth)acrylates. Examples of useful acid functional groups include maleic acid, phosphonic acid, carboxylic acid, sulfonic acid, and mixtures thereof.

Exemplary monomers include diethylene glycol dimethacrylate; triethylene glycol dimethacrylate; tetraethyleneglycol dimethacrylate; glycerol-1,2-dimethacrylate; glycerol-1,3-dimethacrylate; the reaction product of butanediol diglycidyl ester and methacrylic acid; tetrahydrofurfural methacrylate; methacryloxyethyl maleic ester; methacryloxyethyl succinate; urethane dimethacrylate; Bis-GMA; trimethylolpropane tri(meth)acrylate; Ethoxylated bisphenol-A dimethacrylate; bisphenol-A dimethacrylate; and, mixtures thereof. Monomers having a solubility in water higher than 5% are less preferred but still within the scope of the invention. Monomers having a solubility in water less than about 1% are more preferred. Highly water soluble monomers such as hydroxyethyl methacrylate and hydroxypropyl methacrylate tend to provide lower adhesion and are less suitable for use in compositions of the invention.

An example of a useful urethane dimethacrylate is 7,7,9,63,65 hexamethyl-4,13,60,69-tetra-oxo-3, 14, 19, 24, 29, 34, 39, 44, 49, 54, 59, 70-dodecanaoxa-5,12,61,68-tetra-azadoheptacontane-1,72 diyldimethacrylae, also known as urethane diemthacrylate resin).

A volatile solvent is removed after application of the adhesive and/or primer/adhesive to the dentine, enamel, metal or other dental surface. The monomer is preferably less volatile than the solvent.

Examples of useful photoinitiators include those useful in dental applications, including camphorquinone, diaryliodium metal complex salts, chromophore-substituted halomethyl-s-triazines, organo-phosphine oxide (preferably α-cleavage type phosphineoxide), and halomethyl oxadiazoles, and others conventional in the art, such as those disclosed in U.S. Pat. Nos. 5,595,487, 4,514,342 and 4,514,343 as well as mixtures thereof. An effective amount of such an initiator is employed, as will hereinafter be exemplified.

The activator component preferably comprises from about 0.1 to about 10, more preferably about 0.5 to about 5, more preferably still about 0.5 to about 3, percent by weight of a sulfinate salt and from about 90 to about 99.9, more preferably from about 95 to about 99.5, even more preferably about 97 to about 99.5, percent by weight of an activator component solvent, and optionally a reducing agent such as N,N-bis-(2-hydroxyethyl)-p-toluidine (DHEPT). Examples of useful activator component solvents include acetone, ethanol, water, dimethyl sulfoxide (DMSO), methylene chloride, chloroform, and the like, as well as mixtures thereof.

Preferred sulfinate salts are aromatic, having the general structure 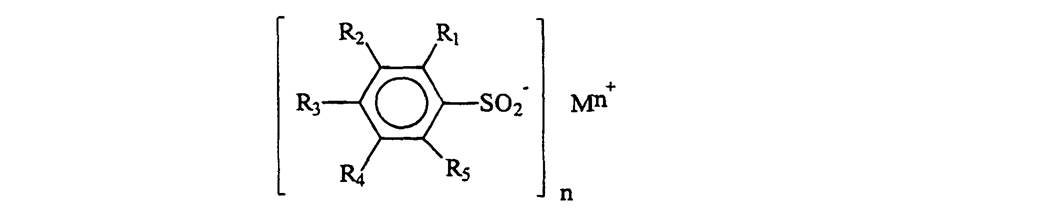 wherein R₁-R₅ are individually a hydrogen or an alkyl group having from 1 to about 6 carbon atom, preferably hydrogen or a methyl group in the para-position; M is a cation; and, "n" is an integer being 1 to 4. Preferred examples of Mⁿ⁺ are sodium, calcium, ammonium, potassium or lithium. Para-toluene sodium or lithium sulfinate and benzene sodium or lithium sulfinate are preferred salts.

The dental adhesive composition as described hereinabove, is employed in inventive methods for restoring teeth. A method according to the invention, for adhering an amalgam or non-amalgam dental restorative to a dental surface, includes: (a) preparing the dental surface for restoration; (b) optionally etching the surface with an acid; (c) applying a mixture of an adhesive component and an activator component to the prepared surface, thereby forming a coated dental surface; and, (d) applying an amalgam or non-amalgam dental restorative to the coated dental surface. As an alternative, in step (c), the adhesive and the activator components may be applied sequentially instead of being first mixed. It will also be appreciated that the order of the method steps according to the invention may be varied as appropriate. The inventive methods and compositions are useful with direct and indirect dental procedures. Further, in step (a), it is understood that "preparing the dental surface for restorations" includes preparing the surface for both direct and indirect restorations without limitation.

The step of preparing the dental surface, which may be a cavity or the like, includes conventional techniques such as removing decayed or otherwise damaged portions by mechanical, abrasive, laser or other means. The surface can then be cleaned by washing or other techniques.

To enhance mechanical retention of the restorative material to the dental surfaces, the tooth surface may optionally be etched whereas the indirect restoration surface may be sandblasted and/or silanated and/or acid etched where applicable according to conventional techniques. Any other conventional preparation technique is also within the scope of the invention. Etching is well known in the art, and any etching technique is also within the scope of the invention. For example, etching may be completed by applying an acid such as phosphoric acid to the prepared dental surface. The etching acid is normally removed by washing and/or drying.

The mixture of an adhesive component and an activator component as used in the inventive method, is selected from those inventive dental compositions as discussed hereinabove. It is applied to the surface to provide a coated dental surface.

If employing a non-amalgam type restorative material, such as Prisma TPH Spectrum composite or EnFOrce WF Composite Resin Luting Cement, both available from Dentsply International Inc. (L.D. Caulk Division), the restorative may be applied to the coated dental surface. If the restorative is an amalgam material, such as Dispersalloy (admixed alloy) or Megalloy (spherical alloy) both available from Dentsply International Inc. (L.D. Caulk Division), it is sometimes optionally useful to first apply a chemically curable adhesive to the coated dental surface followed by application of the amalgam restorative. Preferred chemically curable adhesives include for example, Amalgam Bonding Base and Catalyst available from Dentsply International Inc. (L.D. Caulk Division). Such chemically curable adhesives contain an oxidizing agent such as benzoyl peroxide, and a reducing agent such as N,N-bis-(2-hydroxyethyl)-p-toluidine (DHEPT). It will be appreciated that this step of applying a chemically curable adhesive when using an amalgam restorative is preferred but is optional.

### General Experimental

In order to exemplify the inventive compositions, a dental composition as above was prepared. The activator component was a substantially homogeneous mixture of 0.6 percent by weight of para-toluene sodium sulfinate, 79.5 percent by weight of dimethyl ketone and 19.9 percent by weight of ethanol, to form 100 percent by weight of the activator component. The adhesive component was Prime & Bond® 2.1 (abbreviated P&B below) from Dentsply International Inc., known to contain about 50-80 percent by weight of solvent, about 5-15 percent by weight of multifunctional polymerizable compounds having at least three (meth)acrylate moieties and a phosphate moiety, and the balance being other (meth)acrylate monomers and oligomers, one or more fluoride release compounds, and other additives. The activator and adhesive components were used in approximately a 1:1 mixture by volume, and this mixture is discussed below as the "inventive composition".

### Example 1

This inventive composition was tested for shear bond strength (SBS) using a Single Plane Shear Test Assembly apparatus (SPSTA) as described by L.G. Watanabe, et al., in Journal of Dental Research, 76 (Special Edition) Abs. 1398, 1977 and/or other standard compressive shear bond strength test methods as described in U.S. Pat. No. 5,645,429 which is hereby incorporated by reference for such disclosure, and for example, in Dental Materials, vol. 3;185, 1987 by Hammesfahr et al. The compositions tested included self curing (SC) and light curing (LC or VLC for "visible light cure") materials. Further, the inventive materials were tested with etching or non-etching to dental surfaces as will be noted below, and were also compared to various commercially available adhesive products. As will be shown in the following tables, the inventive material shows comparable or improved results over the commercially available products. "SD" as used below is standard deviation, and "Del." is dentin or enamel delamination. Cohesive failure as reported indicate bond fracture occurred within either tooth substrate dentin or enamel or restoratives and are reported as the number of failures (F) per the total number tested (T) (F/T). Both immediate (15 minute) and 24-hour bond strength of different composite resin luting cements to human dentin using the inventive composition or other commercially available dual-cure adhesive systems were evaluated by SPSTA test methods as described above. The results are summarized in TABLE I.

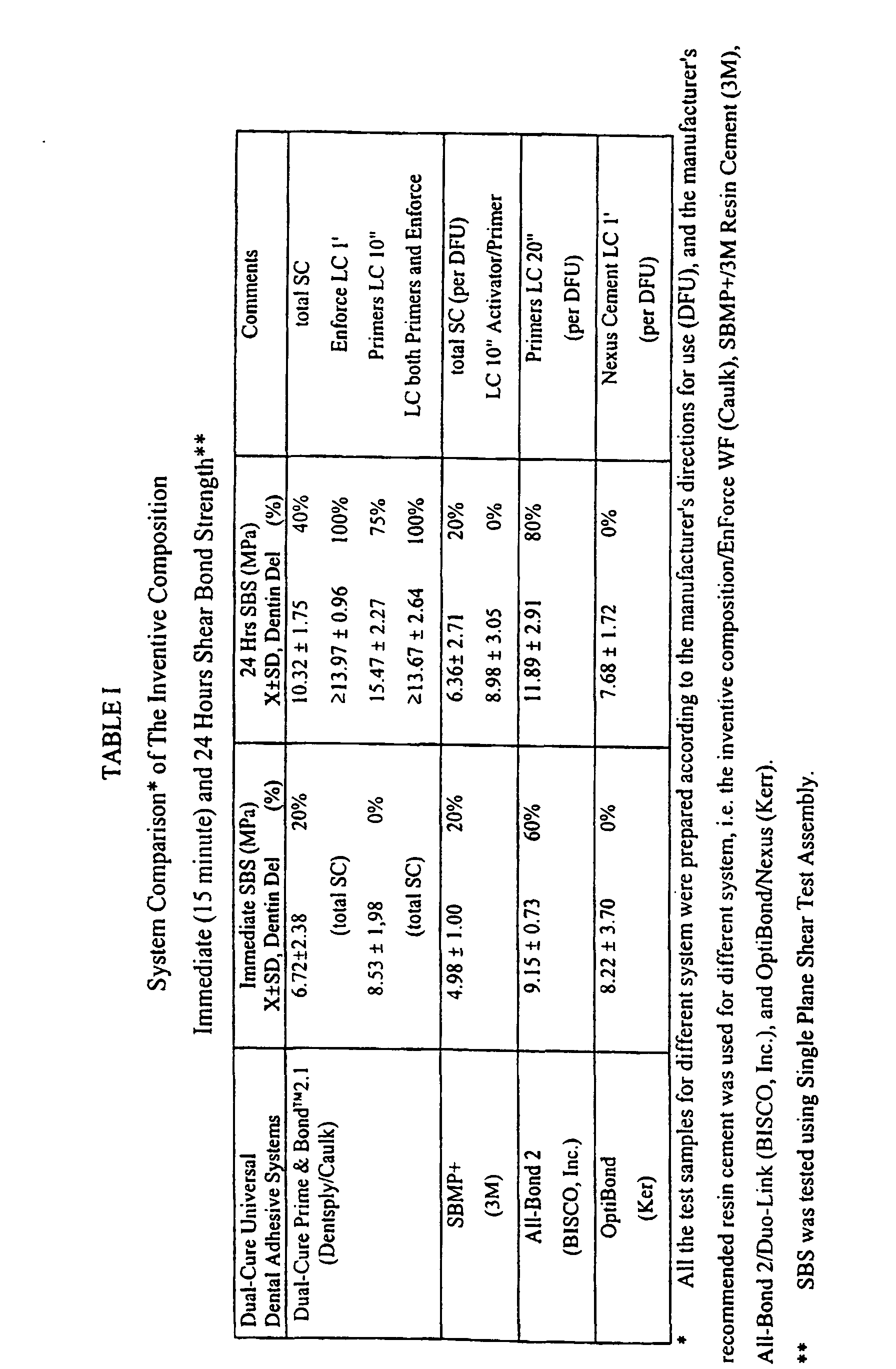

As indicated by the SBS data, the inventive composition, two-bottle dual-cure dental adhesive system showed equivalent (or improved) bonding performance to competitors' multi-bottle dental adhesive system (3M's SBMP+, Bisco's All-Bond and Kerr's OptiBond), with the additional advantages of simplicity, i.e. simple technique, less total application time, time/cost saving, fluoride-release, thin-film thickness, and the like.

### Example II

Using the SPSTA test method, bond strength of different restorative materials to human teeth (dentin) using the inventive composition was evaluated and the results are shown in TABLE II.

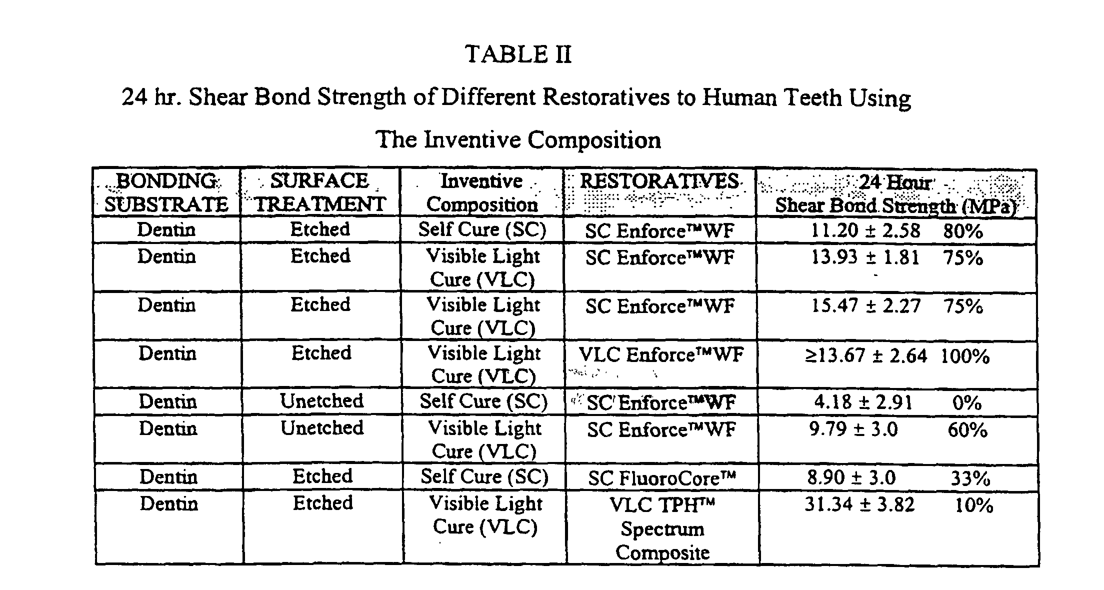

As can be seen from the SBS results in TABLE II (and TABLE III below) the inventive composition can be used to adhere different restorative materials to tooth structure in a variety of direct and indirect restoration applications with good bonding performance.

### Example III

Using the SBS test method as described in Dental Materials, Vol. 3;185, 1987, and in U.S. Pat. No. 5,645,429, 24-hour enamel SBS of different restoratives used in direct and/or indirect restorations using the inventive composition was evaluated. The results are summarized in TABLE III.

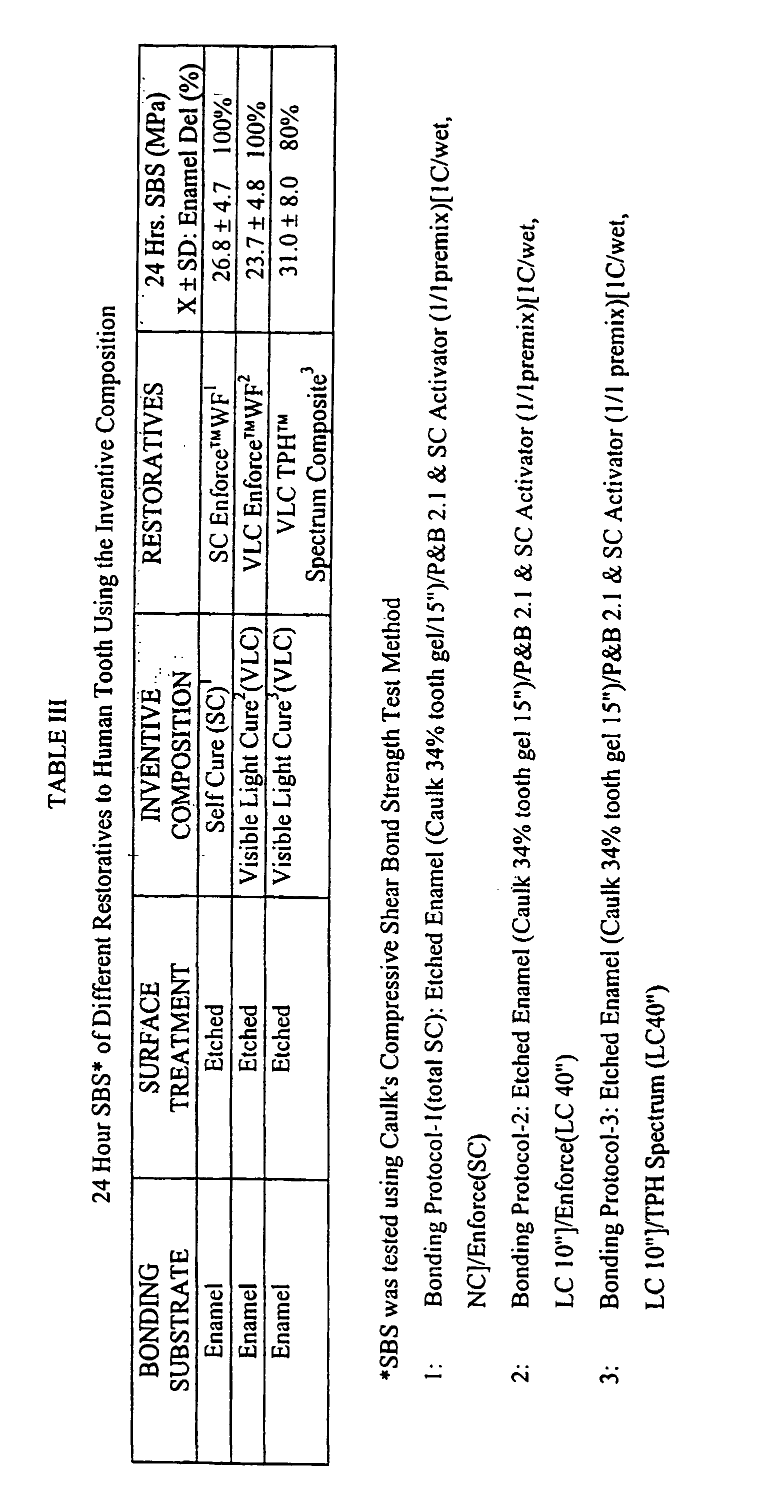

As indicated by the 24 hour enamel SBS data in TABLE III, the inventive composition as a 2-bottle dual cure dental adhesive system showed very good bonding performance (up to 31 MPa, with 140% cohesive failure within human enamel) to human enamel (even in total self cure mode) in relevant to direct and indirect dental applications.

### Example IV

Using the SBS test method as in Example III, except that a porcelain substrate is used in place of human enamel, 24 hour SBS of Enforce Resin Cement to an all-ceramic substrate (commercially available from DENTSPLY International Inc.) using the inventive composition was tested. The obtained results are shown in TABLE IV, indicated that the inventive composition can be used to adhere to a porcelain substrate with high bond strength.

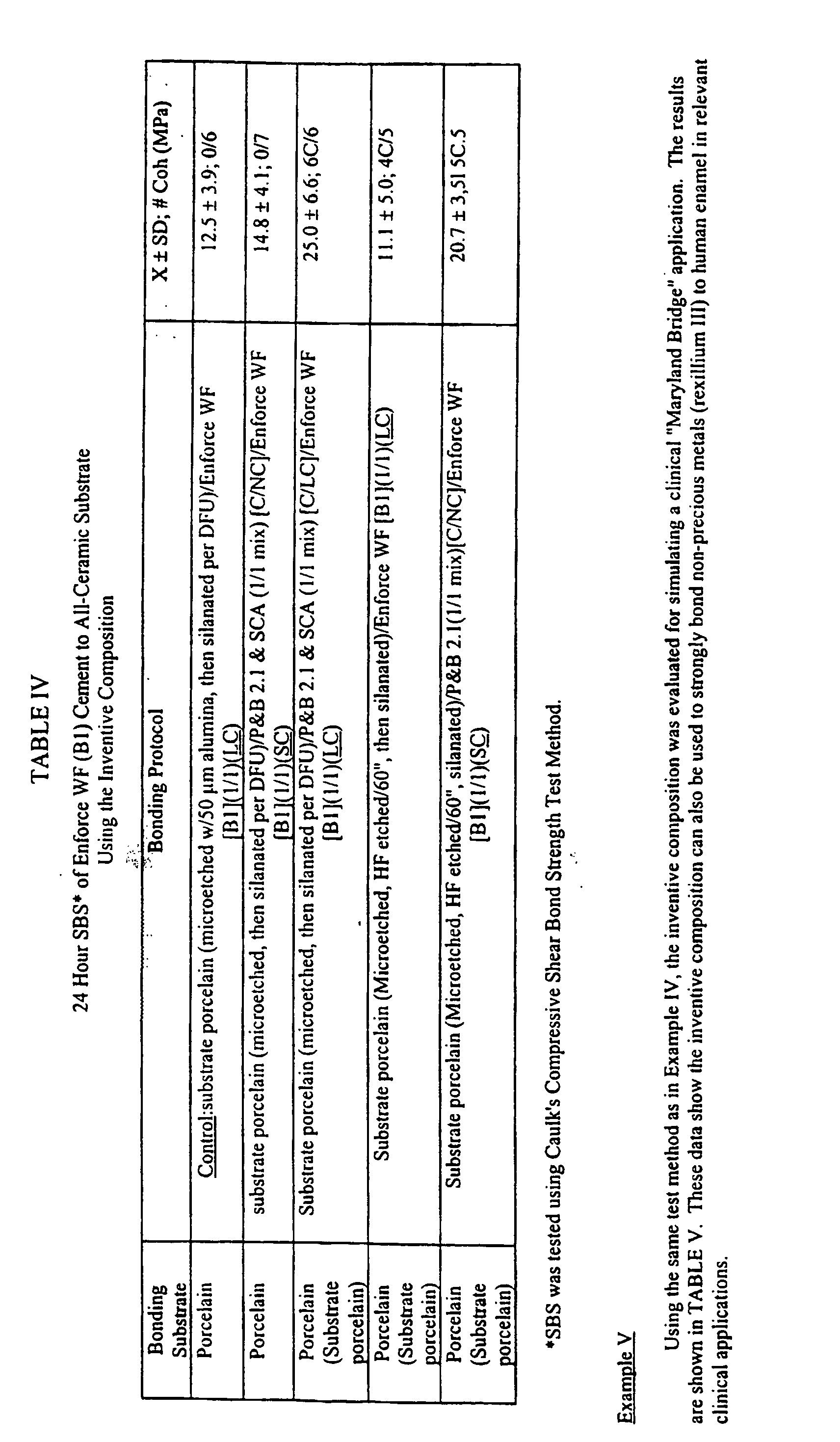

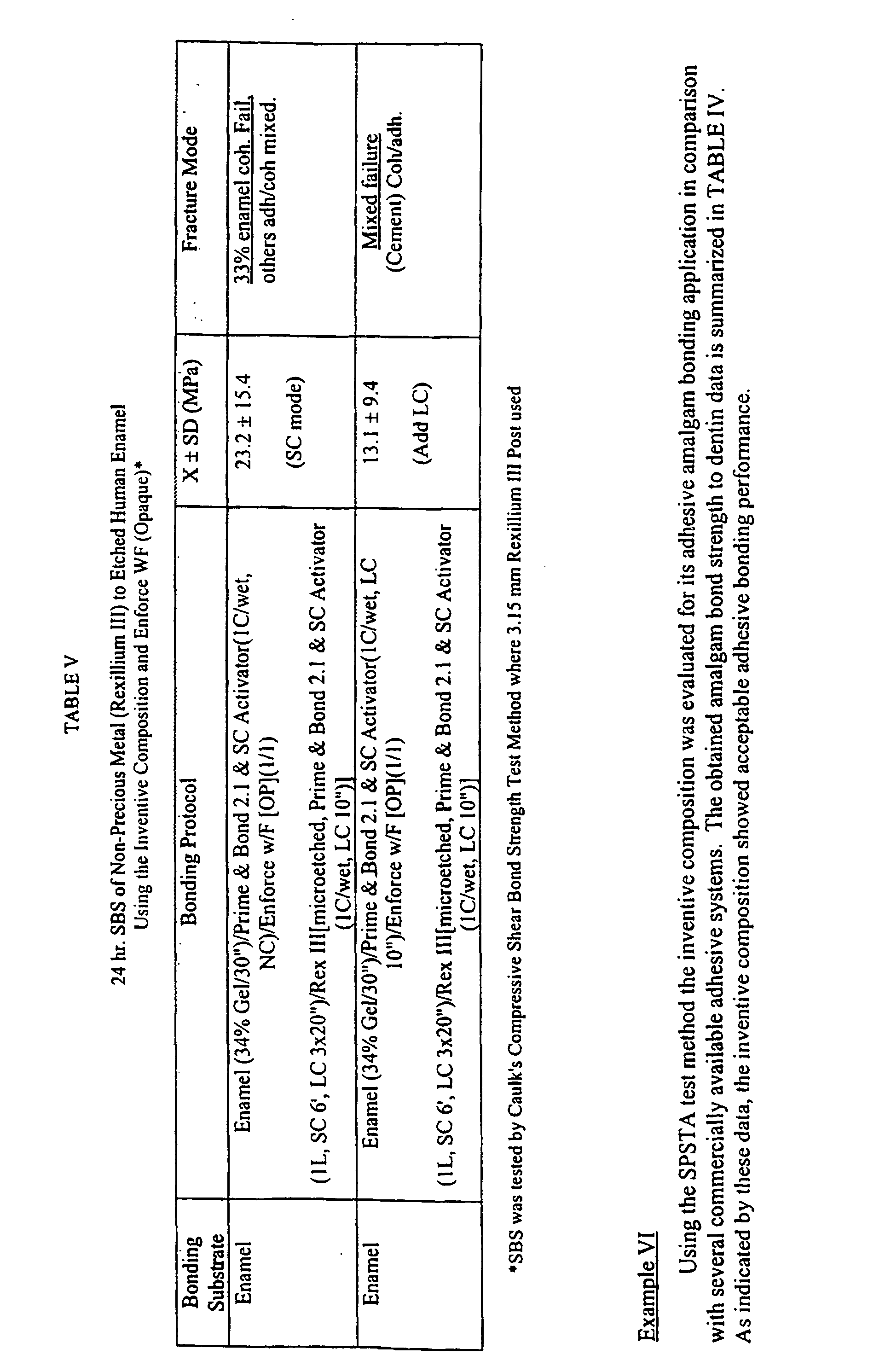

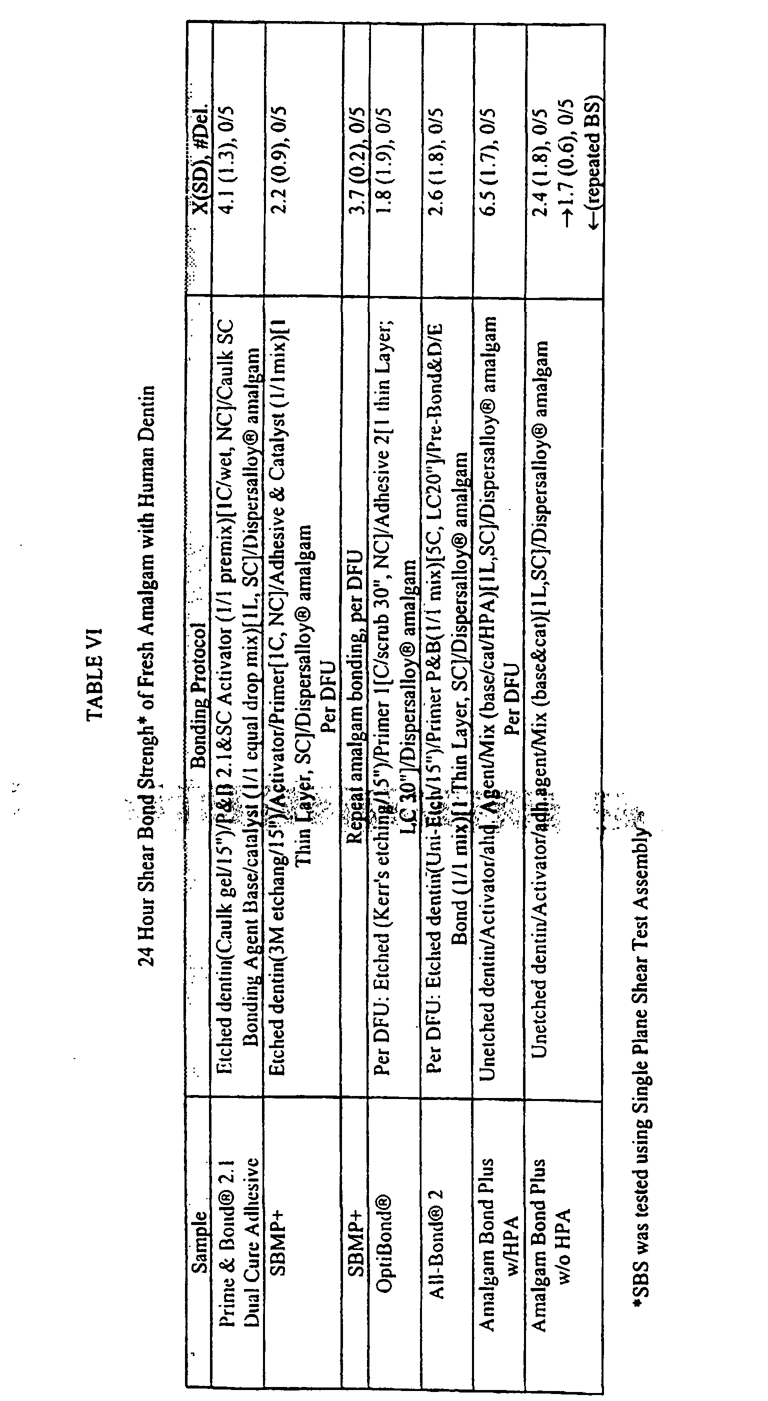

It is apparent therefore, that the dental compositions as described herein are effective in carrying out the objects of the invention. While the principles of the invention have been made clear by the illustrative embodiments discussed, those skilled in the art will appreciate that modifications to composition components including the addition of other components to the composition for promoting chemical or photocure polymerization, amounts, grades, process and method conditions and the like, can be made within the scope of the invention.

## Claims

1. A dental adhesive composition comprising
(a) 5 to 80 percent by weight of an adhesive component comprising
(a1) at least 35 percent by weight of a volatile organic solvent component,
(a2) at least 5 percent by weight of one or more polymerizable (meth)acrylate compounds selected from the group consisting of multifunctional polymerizable compounds having at least three (meth)acrylate moieties and a phosphate moiety,
(a3) an effective amount of a polymerization initiator and
(a4) optionally a filler component selected from the group consisting of silanated inorganic silica; a sol-gel inorganic/organic nano-scale composite, and a prepolymerized organic filler, and
(b) 20 to 95 percent by weight of an activator component comprising
(b1) 0.1 to 10 percent by weight of an aromatic sulfinate salt selected from para-toluene sodium sulfinate, para-toluene lithium sulfinate, benzene sodium sulfinate, benzene lithium sulfinate or mixtures thereof, and
(b2) 90 to 99.9 percent by weight of an activator component solvent.

2. A composition as in claim 1, wherein said polymerizable (meth)acrylate compound is substantially soluble in said volatile organic solvent component.

3. A composition as in claim 1, wherein said volatile organic solvent component is selected from the group consisting of ethanol, methanol, isopropanol, dimethyl ketone, ethylmethyl ketone, water, and mixtures thereof.

4. A composition as in claim 1, further comprising a fluoride release agent.

5. A composition as in claim 1, further comprising a cure additive selected from the group consisting of self-cure and light cure additives and mixtures thereof, such that said additive promotes cure interactions between the composition and a restorative material.

6. A composition as in claim 1, wherein said activator component solvents is selected from the group consisting of acetone, ethanol, water, dimethyl sulfoxide (DMSO), methylene chloride, chloroform, and mixtures thereof.

7. A composition as in claim 1, wherein said initiator is a photoinitiator.

8. A composition as in claim 7, wherein said photoinitiator is selected from the group consisting of camphorquinone, diaryliodium metal complex salts, chromophore-substituted halomethyl-s-triazines, phosphine oxide, halomethyl oxadiazoles, and mixtures thereof.

## Patentansprüche

1. Dentalkleberzusammensetzung, die aufweist:
(a) 5 bis 80 Gew.-% einer Kleberkomponente, die aufweist
(a1) mindestens 35 Gew.% einer flüchtigen organischen Lösungsmittelkomponente,
(a2) mindestens 5 Gew.-% einer oder mehrerer polymerisierbarer (Meth)acrylatverbindung(en), ausgewählt aus der Gruppe bestehend aus multifunktionellen polymerisierbaren Verbindungen mit mindestens drei (Meth)acrylat-Einheiten und einer Phosphat-Einheit,
(a3) eine wirksame Menge eines Polymerisationsinitiators, und
(a4) gegebenenfalls eine Füllstoffkomponente, ausgewählt aus der Gruppe bestehend aus einer silanisierten anorganischen Silikamasse; einem Sol/Gelanorganischen/organischen-Nanoschuppen-Verbundstoff, und einem prepolymerisierten organischen Füllstoff, und
(b) 20 bis 95 Gew.-% eines Aktivators, der aufweist:
(b1) 0,1 bis 10 Gew.-% eines aromatischen Sulfinatsalzes, ausgewählt aus Natriumparatoluolsulfinat, Lithiumparatoluolsulfonat, Natriumbenzolsulfinat, Lithiumbenzolsulfinat oder Mischungen davon, und
(b2) 90 bis 99,9 Gew.-% eines Aktivator-Lösungsmittels.

2. Zusammensetzung nach Anspruch 1, worin die polymerisierbare (Meth)acrylatverbindung in der flüchtigen organischen Lösungsmittelkomponente im wesentlichen löslich ist.

3. Zusammensetzung nach Anspruch 1, worin die flüchtige organische Lösungsmittelkomponente ausgewählt ist aus der Gruppe bestehend aus Ethanol, Methanol, Isopropanol, Dimethylketon, Ethylmethylketon, Wasser, und Mischungen davon.

4. Zusammensetzung nach Anspruch 1, die ferner ein Fluorid-Freisetzungsmittel aufweist.

5. Zusammensetzung nach Anspruch 1, die ferner ein Härtungsmittel aufweist, ausgewählt aus der Gruppe bestehend aus selbsthärtenden und lichthärtenden Mitteln und Mischungen davon, wobei das Mittel die gegenseitige Härtungswirkung zwischen der Zusammensetzung und einem Restaurierungsmaterial fördert.

6. Zusammensetzung nach Anspruch 1, worin das Aktivator-Lösungsmittel ausgewählt ist aus der Gruppe bestehend aus Aceton, Ethanol, Wasser, Dimethylsulfoxid (DMSO), Methylenchlorid, Chloroform, und Mischungen davon.

7. Zusammensetzung nach Anspruch 1, worin der Initiator ein Photoinitiator ist.

8. Zusammensetzung nach Anspruch 7, worin der Photoinitiator ausgewählt ist aus der Gruppe bestehend aus Campherchinon, Diaryliodium-Metallkomplex-Salzen, Chromophor-substituierten Halogenmethyl-s-triazinen, Phosphinoöxid, Halogenmethyloxadiazolen, und Mischungen davon.

## Revendications

1. Composition d'adhésif dentaire comprenant
(a) 5 à 80 % en poids d'un constituant adhésif comprenant
(a1) au moins 35 % en poids d'un solvant organique volatile,
(a2) au moins 5 % en poids d'un ou plusieurs composés (méth)acrylate polymérisables choisis dans le groupe consistant en des composés polymérisables multifonctionnels ayant au moins trois groupements (méth)acrylate et un groupement phosphate,
(a3) une quantité efficace d'un initiateur de polymérisation et (a4) facultativement, un constituant servant de charge choisi dans le groupe consistant en une silice inorganique silanée ; un composite à nanoéchelle inorganique/organique sol-gel et une charge organique prépolymérisée, et
(b) 20 à 95 % en poids d'un constituant activateur comprenant
(b1) 0,1 à 10 % en poids d'un sel sulfinate aromatique choisi entre le paratoluènesulfinate de sodium, le paratoluènesulfinate de lithium, le benzènesulfinate de sodium, le benzènesulfinate de lithium ou leurs mélanges et
(b2) 90 à 99,9 % en poids d'un solvant du constituant activateur.

2. Composition suivant la revendication 1, dans laquelle le composé (méth)acrylate polymérisable est substantiellement soluble dans ledit solvant organique volatile.

3. Composition suivant la revendication 1, dans laquelle ledit solvant organique volatile est choisi dans le groupe consistant en l'éthanol, le méthanol, l'isopropanol, la diméthylcétone, l'éthylméthylcétone, l'eau et leurs mélanges.

4. Composition suivant la revendication 1, comprenant en outre un agent libérant des fluorures.

5. Composition suivant la revendication 1, comprenant en outre un additif de durcissement choisi dans le groupe consistant en des additifs d'autodurcissement et des additifs de photodurcissement et leurs mélanges, de telle sorte que ledit additif favorise les interactions de durcissement entre la composition et un matériau de reconstitution.

6. Composition suivant la revendication 1, dans laquelle ledit solvant du constituant activateur est choisi dans le groupe consistant en l'acétone, l'éthanol, l'eau, le diméthylsulfoxyde (DMSO), le chlorure de méthylène, le chloroforme et leurs mélanges.

7. Composition suivant la revendication 1, dans laquelle ledit initiateur est un photoinitiateur.

8. Composition suivant la revendication 7, dans laquelle ledit photoinitiateur est choisi dans le groupe consistant en la camphoquinone, des sels complexes métalliques de diaryliodium, des halogénométhyl-s-triazines substituées avec un chromophore, l'oxyde de phosphine, des halogénométhyloxadiazoles et leurs mélanges.
